# EUROPEAN PATENT SPECIFICATION

(11) **EP 1 448 778 B1**
(45) Date of publication and mention of the grant of the patent: **07.12.2011**
(21) Application number: 02779424.7
(22) Date of filing: 26.09.2002
(51) Int. Cl.: C12N 15/52, C12P 13/00, C12P 13/08

(54) **PROCESS FOR THE PREPARATION OF NON-AROMATIC L-AMINO ACIDS USING STRAINS OF THE ENTEROBACTERIACEAE FAMILY**
VERFAHREN ZUR HERSTELLUNG VON NICHTAROMATISCHEN L-AMINOSÄUREN DURCH STÄMME AUS DER FAMILE DER ENTEROBACTERIACEAE
PROCEDE DE PREPARATION D'ACIDES L-AMINES NON AROMATIQUES UTILISANT DES SOUCHES DE LA FAMILLE DES ENTEROBACTERIACEAE

(30) Priority: 24.11.2001 DE 10157721
(43) Date of publication of application: 25.08.2004
(73) Proprietor: Evonik Degussa GmbH, 45128 Essen (DE)
(72) Inventor: RIEPING, Mechthild, 33619 Bielefeld (DE)
(86) International application number: PCT/EP2002/010792
(87) International publication number: WO 2003/046184

(56) References cited:
- WO-A-02/29080
- WO-A-95/04074
- WO-A-99/02721
- US-A- 4 278 765
- JETTEN M S M ET AL: "RECENT ADVANCES IN THE PHYSIOLOGY AND GENETICS OF AMINO ACID-PRODUCING BACTERIA" CRC CRITICAL REVIEWS IN BIOTECHNOLOGY, CRC PRESS, BOCA RATON, FL, US, vol. 15, no. 1, 1995, pages 73-103, XP000613291 ISSN: 0738-8551
- KRAEMER REINHARD: "Genetic and physiological approaches for the production of amino acids" JOURNAL OF BIOTECHNOLOGY, ELSEVIER SCIENCE PUBLISHERS, AMSTERDAM, NL, vol. 45, no. 1, 1996, pages 1-21, XP002178648 ISSN: 0168-1656
- TATARKO M., ROMEO T.: "Disruption of a global regulatory gene to enhance central carbon flux into phenylalanine biosynthesis in Escherichia coli" CURR. MICROBIOL., vol. 43, July 2001 (2001-07), pages 26-32, XP002229632
- ROMEO T.: "Global regulation by the small RNA-binding protein CsrA and the non-coding molecule CsrB." MOL. MICROBIOL., vol. 29, no. 6, September 1998 (1998-09), pages 1321-1330, XP002229633
- LIU ET AL: "The RNA molecule CsrB binds to the global regulatory protein CsrA and antagonizes its activity in Escherichia coli" PROCEEDINGS OF THE NATIONAL ACADEMY OF SCIENCES OF USA, NATIONAL ACADEMY OF SCIENCE. WASHINGTON, US, vol. 272, no. 28, 11 July 1997 (1997-07-11), pages 17502-17510, XP002085763 ISSN: 0027-8424

## Description

### Field of the Invention

This invention relates to a process for the preparation of non-aromatic L-amino acids, in particular L-threonine, using strains of the Enterobacteriaceae family in which the csrA gene is enhanced.

### Prior Art

L-Amino acids, in particular L-threonine, are used in human medicine and in the pharmaceuticals industry, in the foodstuffs industry and very particularly in animal nutrition.

It is known that L-amino acids are prepared by fermentation of strains of Enterobacteriaceae, in particular Escherichia coli (E. coli) and Serratia marcescens. Because of their great importance, work is constantly being undertaken to improve the preparation processes. Improvements to the process can relate to fermentation measures, such as e.g. stirring and supply of oxygen, or the composition of the nutrient media, such as e.g. the sugar concentration during the fermentation, or the working up to the product form, by e.g. ion exchange chromatography, or the intrinsic output properties of the microorganism itself.

Methods of mutagenesis, selection and mutant selection are used to improve the output properties of these microorganisms. Strains which are resistant to antimetabolites, such as e.g. the threonine analogue α-amino-β-hydroxyvaleric acid (AHV), or are auxotrophic for metabolites of regulatory importance and produce L-amino acid, such as e.g. L-threonine, are obtained in this manner.

Methods of the recombinant DNA technique have also been employed for some years for improving the strain of strains of the Enterobacteriaceae family which produce L-amino acids, by amplifying individual amino acid biosynthesis genes and investigating the effect on the production.

The csrA gene has been described by Romeo et al. (Journal of Bacteriology 175(15), 4744-4755 (1993)). It is the subject of numerous studies. US-A-6, 268, 471 shows the amino acid sequence of the CsrA polypeptide and describes the action on the formation of enzymes of glycogen biosynthesis, gluconeogenesis and glycolysis. WO99/02721 describes csrB-RNA and its action on the expression of the CsrA protein. WO00/73484 demonstrates that by lowering the amount of functional CsrA protein available, production of the aromatic amino acids phenylalanine, tyrosine and tryptophan can be improved.

### Object of the Invention

The inventors had the object of providing new measures for improved preparation of L-amino acids, in particular L-threonine.

### Summary of the Invention

The invention provides a process for the preparation of non-aromatic L-amino acids, in particular L-threonine, using microorganisms of the Enterobacteriaceae family which, in particular, already produce L-amino acids and in which the nucleotide sequence in particular endogenous nucleotide sequence, which codes for the csrA gene is overexpressed.

### Detailed Description of the invention

"Endogenous genes" or "endogenous nucleotide sequences" are understood as meaning the genes or nucleotide sequences present in the population of a species.

Where L-amino acids or amino acids are mentioned in the following, this means one or more amino acids, including their salts, chosen from the group consisting of L-valine, L-lysine and L-homoserine L-Threonine is particularly preferred.

The term "enhancement" in this connection describes the increase in the intracellular activity of one or more enzymes or proteins in a microorganism which are coded by the corresponding DNA, for example by increasing the number of copies of the gene or genes, using a potent promoter or a gene or allele which codes for a corresponding enzyme or protein with a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The invention provides a process for the preparation of L-amino acids, in particular L-threonine, characterized in that the following steps are carried out:
a) fermentation of the microorganisms of the Enterobacteriaceae family, in particular of the genus Escherichia, which produce the desired L-amino acid and in which the csrA gene or nucleotide sequences which code for it are over-expressed.

The crsA gene and optionally alleles of the csrA gene are over-expressed under conditions which are suitable for formation of the CsrA protein.

Further steps are:
b) concentration of the desired L-amino acid in the medium or in the cells of the microorganisms, and
c) isolation of the desired L-amino acid, constituents of the fermentation broth and/or the biomass in its entirety or portions (≥ 0 to 100 %) thereof optionally remaining in the product.

The microorganisms which the present invention provides can produce L-amino acids from glucose, sucrose, lactose, fructose, maltose, molasses, optionally starch, optionally cellulose or from glycerol and ethanol. They are representatives of the Enterobacteriaceae family chosen from the genera Escherichia, Erwinia, Providencia and Serratia. The genera Escherichia and Serratia are preferred. Of the genus Escherichia the species Escherichia coli and of the genus Serratia the species Serratia marcescens are to be mentioned in particular.

Suitable strains, which produce L-threonine in particular, of the genus Escherichia, in particular of the species Escherichia coli, are, for example
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132.

Suitable L-threonine-producing strains of the genus Serratia, in particular of the species Serratia marcescens, are, for example
Serratia marcescens HNr21
Serratia marcescens TLr156
Serratia marcescens T2000.

Strains from the Enterobacteriaceae family which produce L-threonine preferably have, inter alia, one or more genetic or phenotypic features chosen from the group consisting of: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to rifampicin, resistance to valine analogues, such as, for example, valine hydroxamate, resistance to purine analogues, such as, for example, 6-dimethylaminopurine, a need for L-methionine, optionally a partial and compensable need for L-isoleucine, a need for meso-diaminopimelic acid, auxotrophy in respect of threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally an ability for sucrose utilization, enhancement of the threonine operon, enhancement of homoserine dehydrogenase I-aspartate kinase I, preferably of the feed back resistant form, enhancement of homoserine kinase, enhancement of threonine synthase, enhancement of aspartate kinase, optionally of the feed back resistant form, enhancement of aspartate semialdehyde dehydrogenase, enhancement of phosphoenol pyruvate carboxylase, optionally of the feed back resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic acid formation.

It has been found that microorganisms of the Enterobacteriaceae family produce said non-aromatic L-amino acids, in particular L-threonine, in an improved manner after over-expression, of the csrA gene.

The nucleotide sequences of the genes of Escherichia coli belong to the prior art and can also be found in the genome sequence of Escherichia coli published by Blattner et al. (Science 277: 1453-1462 (1997)).

The csrA gene is described, inter alia, by the following data:

| | |
|---|---|
| Descriptions: | Regulator CsrA (carbon storage regulator A), RNA-binding protein, global regulatory function, controls glycogen synthesis, gluconeogenesis, cell size and surface properties |
| Reference: | Romeo et al.; Journal of Bacteriology 175 (15): 4744-4755 (1993); Liu and Romeo; Journal of Bacteriology 179 (14): 4639-4642 (1997) |
| Accession No.: | AE000353 |

The nucleic acid sequences can be found in the databanks of the National Center for Biotechnology Information (NCBI) of the National Library of Medicine (Bethesda, MD, USA), the nucleotide sequence databank of the European Molecular Biology Laboratories (EMBL, Heidelberg, Germany or Cambridge, UK) or the DNA databank of Japan (DDBJ, Mishima, Japan).

The gene described in the text reference stated can be used according to the invention. Alleles of the gene which result from the degeneracy of the genetic code or due to "sense mutations" of neutral function can furthermore be used.

To achieve an enhancement, for example, expression of the gene or the catalytic properties of the protein can be increased. The two measures can optionally be combined.

To achieve an over-expression, the number of copies of the corresponding genes can be increased, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative L-threonine production. The expression is likewise improved by measures to prolong the life of the m-RNA. Furthermore, the enzyme activity is also increased by preventing the degradation of the enzyme protein. The genes or gene constructs can either be present in plasmids with a varying number of copies, or can be integrated and amplified in the chromosome. Alternatively, an over-expression of the genes in question can furthermore be achieved by changing the composition of the media and the culture procedure.

The expert can find instructions in this respect, inter alia, in Chang and Cohen (Journal of Bacteriology 134: 1141-1156 (1978)), in Hartley and Gregori (Gene 13: 347-353 (1981)), in Amann and Brosius (Gene 40: 183-190 (1985)), in de Broer et al. (Proceedings of the National Academy of Sciences of the United States of America 80: 21-25 (1983)), in LaVallie et al. (BIO/TECHNOLOGY 11: 187-193 (1993)), in PCT/US97/13359, in Llosa et al. (Plasmid 26: 222-224 (1991)), in Quandt and Klipp (Gene 80: 161-169 (1989)), in Hamilton (Journal of Bacteriology 171: 4617-4622 (1989)), in Jensen and Hammer (Biotechnology and Bioengineering 58: 191-195 (1998)) and in known textbooks of genetics and molecular biology.

Plasmid vectors which can replicate in Enterobacteriaceae, such as e.g. cloning vectors derived from pACYC184 (Bartolomé et al.; Gene 102: 75-78 (1991)), pTrc99A (Amann et al.; Gene 69: 301-315 (1988)) or pSC101 derivatives (Vocke and Bastia, Proceedings of the National Academy of Sciences USA 80(21): 6557-6561 (1983)) can be used. A strain transformed with a plasmid vector where the plasmid vector carries at least the csrA gene or nucleotide sequences which code for this can be employed in a process according to the invention.

It is also possible, at the same time or optionally separately, to achieve an over-expression of the CsrA regulator by attenuation of the csrB gene.

The term "attenuation" describes the reduction or elimination of the intracellular activity of one or more enzymes (proteins) or ribonucleic acids (RNAs) in a microorganism which are coded by the corresponding DNA, for example by using a weak promoter or a gene or allele which codes for a corresponding enzyme or a corresponding ribonucleic acid with a low activity or inactivates the corresponding enzyme (protein), gene or ribonucleic acid, and optionally combining these measures.

By attenuation measures, the activity or concentration of the corresponding protein or of the corresponding ribonucleic acid is in general reduced to 0 to 75%, 0 to 50%, 0 to 25%, 0 to 10% or 0 to 5% of the activity or concentration of the wild-type protein or of the wild-type RNA, or of the activity or concentration of the protein or of the RNA in the starting microorganism.

Finally, it is also possible to transfer mutations which affect the expression or activity of the particular genes into various strains by sequence exchange (Hamilton et al. (Journal of Bacteriology 171: 4617-4622 (1989)), conjugation or transduction.

It may furthermore be advantageous for the production of L-threonine, with strains of the Enterobacteriaceae family to enhance one or more enzymes of the known threonine biosynthesis pathway or enzymes of anaplerotic metabolism or enzymes for the production of reduced nicotinamide adenine dinucleotide phosphate or enzymes of glycolysis or PTS enzymes or enzymes of sulfur metabolism, in addition to the enhancement of the csrA gene. The use of endogenous genes is in general preferred.

Thus, for example, at the same time one or more of the genes chosen from the group consisting of
- the thrABC operon which codes for aspartate kinase, homoserine dehydrogenase, homoserine kinase and threonine synthase (US-A-4,278,765),
- the pyc gene which codes for pyruvate carboxylase (DE-A-19 831 609),
- the pps gene which codes for phosphoenol pyruvate synthase (Molecular and General Genetics 231: 332-336 (1992)),
- the ppc gene which codes for phosphoenol pyruvate carboxylase (Gene 31: 279-283 (1984)),
- the pntA and pntB genes which code for transhydrogenase (European Journal of Biochemistry 158: 647-653 (1986)),
- the rhtB gene which imparts homoserine resistance (EP-A-0 994 190),
- the mqo gene which codes for malate:quinone oxidoreductase (Journal of Bacteriology 182: 6892-6899 (2000)),
- the rhtC gene which imparts threonine resistance (EP-A-1 013 765),
- the thrE gene of Corynebacterium glutamicum which codes for the threonine export protein (EP-A-1 085 091),
- the gdhA gene which codes for glutamate dehydrogenase (Nucleic Acids Research 11: 5257-5266 (1983); Gene 23: 199-209 (1983)),
- the hns gene which codes for the DNA-binding protein HLP-II (Molecular and General Genetics 212: 199-202 (1988)),
- the lrp gene which codes for the regulator of the leucine regulon (Journal of Biological Chemistry 266: 10768-10774 (1991)),
- the pgm gene which codes for phosphoglucomutase (Journal of Bacteriology 176: 5847-5851 (1994)),
- the fba gene which codes for fructose biphosphate aldolase (Biochemical Journal 257: 529-534 (1989)),
- the ptsH gene of the ptsHIcrr operon which codes for the phosphohistidine protein hexose phosphotransferase of the phosphotransferase system PTS (Journal of Biological Chemistry 262: 16241-16253 (1987)),
- the ptsI gene of the ptsHIcrr operon which codes for enzyme I of the phosphotransferase system PTS (Journal of Biological Chemistry 262: 16241-16253 (1987)),
- the crr gene of the ptsHIcrr operon which codes for the glucose-specific IIA component of the phosphotransferase system PTS (Journal of Biological Chemistry 262: 16241-16253 (1987)),
- the ptsG gene which codes for the glucose-specific IIBC component (Journal of Biological Chemistry 261: 16398-16403 (1986)),
- the mopB gene which codes for 10 Kd chaperone (Journal of Biological Chemistry 261: 12414-12419 (1986)) and is also known by the name groES,
- the ahpC gene of the ahpCF operon which codes for the small sub-unit of alkyl hydroperoxide reductase (Proceedings of the National Academy of Sciences of the United States of America 92: 7617-7621 (1995)),
- the ahpF gene of the ahpCF operon which codes for the large sub-unit of alkyl hydroperoxide reductase (Proceedings of the National Academy of Sciences of the United States of America 92: 7617-7621 (1995)),
- the cysK gene which codes for cysteine synthase A (Journal of Bacteriology 170: 3150-3157 (1988))
- the cysB gene which codes for the regulator of the cys regulon (Journal of Biological Chemistry 262: 5999-6005 (1987)),
- the cysJ gene of the cysJIH operon which codes for the flavoprotein of NADPH sulfite reductase (Journal of Biological Chemistry 264: 15796-15808 (1989), Journal of Biological Chemistry 264: 15726-15737 (1989)),
- the cysI gene of the cysJIH operon which codes for the haemoprotein of NADPH sulfite reductase (Journal of Biological Chemistry 264: 15796-15808 (1989), Journal of Biological Chemistry 264: 15726-15737 (1989)),
- the cysH gene of the cysJIH operon which codes for adenylyl sulfate reductase (Journal of Biological Chemistry 264: 15796-15808 (1989), Journal of Biological Chemistry 264: 15726-15737 (1989)),
- the phoB gene of the phoBR operon which codes for the positive regulator PhoB of the pho regulon (Journal of Molecular Biology 190 (1): 37-44 (1986)),
- the phoR gene of the phoBR operon which codes for the sensor protein of the pho regulon (Journal of Molecular Biology 192 (3): 549-556 (1986)),
- the phoE gene which codes for protein E of the outer cell membrane (Journal of Molecular Biology 163 (4): 513-532 (1983)),
- the pykF gene which codes for fructose-stimulated pyruvate kinase I (Journal of Bacteriology 177 (19): 5719-5722 (1995)),
- the pfkB gene which codes for 6-phosphofructokinase II (Gene 28 (3): 337-342 (1984)),
- the malE gene which codes for the periplasmic binding protein of maltose transport (Journal of Biological Chemistry 259 (16): 10606-10613 (1984)),
- the rseA gene of the rseABC operon which codes for a membrane protein with anti-sigmaE activity (Molecular Microbiology 24 (2): 355-371 (1997)),
- the rseC gene of the rseABC operon which codes for a global regulator of the sigmaE factor (Molecular Microbiology 24 (2): 355-371 (1997)),
- the sodA gene which codes for superoxide dismutase (Journal of Bacteriology 155 (3): 1078-1087 (1983)),
- the sucA gene of the sucABCD operon which codes for the decarboxylase sub-unit of 2-ketoglutarate dehydrogenase (European Journal of Biochemistry 141 (2): 351-359 (1984)),
- the sucB gene of the sucABCD operon which codes for the dihydrolipoyltransouccinase E2 sub-unit of 2-ketoglutarate dehydrogenase (European Journal of Biochemistry 141 (2): 361-374 (1984)),
- the sucC gene of the sucABCD operon which codes for the β-sub-unit of succinyl-CoA synthetase (Biochemistry 24 (22): 6245-6252 (1985)),
- the sucD gene of the sucABCD operon which codes for the α-sub-unit of succinyl-CoA synthetase (Biochemistry 24 (22): 6245-6252 (1985)) and
- the iclR gene which codes for the regulator IclR of central intermediate metabolism (Journal of Bacteriology 172 (5): 2642-2649 (1990)
can be over-expressed.

It may furthermore be advantageous for the production of L-threonine, in addition to the enhancement of the csrA gene, for one or more of the genes chosen from the group consisting of
- the tdh gene which codes for threonine dehydrogenase (Journal of Bacteriology 169: 4716-4721 (1987)),
- the mdh gene which codes for malate dehydrogenase (E.G. 1.1.1.37) (Archives in Microbiology 149: 36-42 (1987)),
- the gene product of the open reading frame (orf) yjfA (Accession Number AAC77180 of the National Center for Biotechnology Information (NCBI, Bethesda, MD, USA)),
- the gene product of the open reading frame (orf) ytfP (Accession Number AAC77179 of the National Center for Biotechnology information (NCBI, Bethesda, MD, USA)),
- the pckA gene which codes for the enzyme phosphoenol pyruvate carboxykinase (Journal of Bacteriology 172: 7151-7156 (1990)),
- the poxB gene which codes for pyruvate oxidase (Nucleic Acids Research 14(13): 5449-5460 (1986)).
- the aceA gene which codes for the enzyme isocitrate lyase (Journal of Bacteriology 170: 4528-4536 (1988)),
- the dgsA gene which codes for the DgsA regulator of the phosphotransferase system (Bioscience, Biotechnology and Biochemistry 59: 256-251 (1995)) and is also known under the name of the mlc gene,
- the fruR gene which codes for the fructose repressor (Molecular and General Genetics 226: 332-336 (1991)) and is also known under the name of the cra gene,
- the rpoS gene which codes for the sigma³⁸ factor (WO 01/05939) and is also known under the name of the katF gene,
- the aspA gene which codes for aspartate ammonium lyase, (aspartase) (Nucleic Acids Research 13(6): 2063-2074 (1985)) and
- the aceB gene which codes for malate synthase A (Nucleic Acids Research 16(19): 9342 (1988)),
to be eliminated or for the expression thereof to be reduced.

This is effected under conditions which are suitable for reduction or elimination of the intracellular activity of the corresponding enzymes.

In addition to enhancement of the csrA gene it may furthermore be advantageous for the production of L-amino acids, in particular L-threonine, to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced according to the invention can be cultured in the batch process (batch culture), the fed batch process (feed process) or the repeated fed batch process (repetitive feed process). A summary of known culture methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and optionally cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 25°C to 45°C, and preferably 30°C to 40°C. Culturing is continued until a maximum of L-amino acids or L-threonine has formed. This target is usually reached within 10 hours to 160 hours.

The analysis of L-amino acids can be carried out by anion exchange chromatography with subsequent ninhydrin derivation, as described by Spackman et al. (Analytical Chemistry, 30: 1190-1206 (1958)) or it can be carried out by reversed phase HPLC, as described by Lindroth et al. (Analytical Chemistry 51: 1167-1174 (1979)).

The process according to the invention is used for the fermentative preparation of L-amino acids L-threonine, L-valine, L-homoserine and L-lysine, in particular L-threonine.

The present invention is explained in more detail in the following with the aid of embodiment examples.

The minimal (M9) and complete media (LB) for Escherichia coli used are described by J.H. Miller (A Short Course in Bacterial Genetics (1992), Cold Spring Harbor Laboratory Press). The isolation of plasmid DNA from Escherichia coli and all techniques of restriction, ligation, Klenow and alkaline phosphatase treatment are carried out by the method of Sambrook et al. (Molecular Cloning - A Laboratory Manual (1989) Cold Spring Harbor Laboratory Press). Unless described otherwise, the transformation of Escherichia coli is carried out by the method of Chung et al. (Proceedings of the National Academy of Sciences of the United States of America 86: 2172-2175 (1989)).

The incubation temperature for the preparation of strains and transformants is 37°C.

### Example 1

### Construction of the expression plasmid pTrc99AcsrA

The csrA gene from E. coli K12 is amplified using the polymerase chain reaction (PCR) and synthetic oligonucleotides. Starting from the nucleotide sequence of the csrA gene in E. coli K12 MG1655 (Accession Number AE000353, Blattner et al. (Science 277: 1453-1462 (1997)), PCR primers are synthesized (MWG Biotech, Ebersberg, Germany).
csrA1: 5' - CTTTCAAGGAGCAAAGAATG - 3' (SEQ ID No. 1)
csrA2: 5' - GATGAGACGCGGAAAGATTAG - 3' (SEQ ID No. 2)

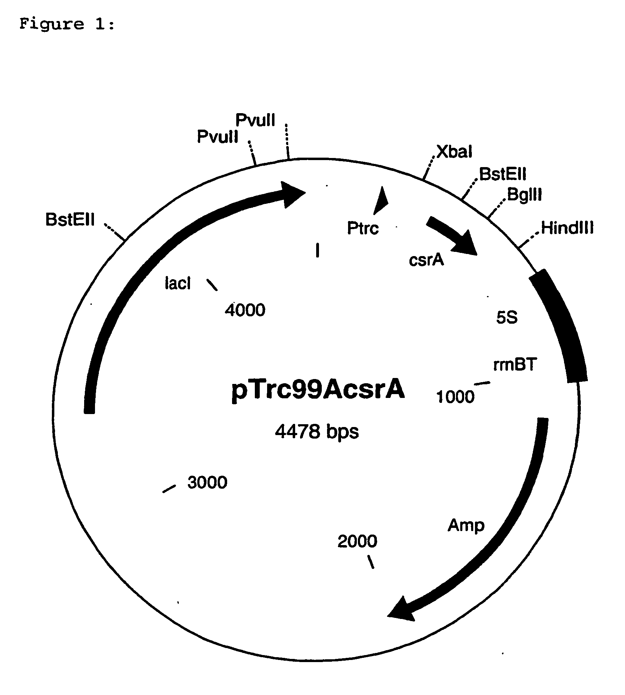

The chromosomal E. coli K12 MG1655 DNA employed for the PCR is isolated according to the manufacturer's instructions with "Qiagen Genomic-tips 100/G" (QIAGEN, Hilden, Germany). A DNA fragment approx. 200 bp in size can be amplified with the specific primers under standard PCR conditions (Innis et al. (1990) PCR Protocols. A Guide to Methods and Applications, Academic Press) with Pfu-DNA polymerase (Promega Corporation, Madison, USA). The PCR product is ligated according to the manufacturer's instructions with the vector pCR-Blunt II-TOPO (Zero Blunt TOPO PCR Cloning Kit, Invitrogen, Groningen, The Netherlands) and transformed into the E. coli strain TOP10. Selection of plasmid-carrying cells takes place on LB agar, to which 50 µg/ml kanamycin are added. After isolation of the plasmid DNA, the vector pCR-Blunt II-TOPO-csrA is cleaved with the restriction enzymes HindIII and XbaI and, after separation in 0.8% agarose gel, the csrA fragment is isolated with the aid of the QIAquick Gel Extraction Kit (QIAGEN, Hilden, Germany). The vector pTrc99A (Pharmacia Biotech, Uppsala, Sweden) is cleaved with the enzymes HindIII and XbaI and ligation is carried out with the csrA fragment isolated. The E. coli strain XL1-Blue MRF' (Stratagene, La Jolla, USA) is transformed with the ligation batch and plasmid-carrying cells are selected on LB agar, to which 50 µg/ml ampicillin are added. Successful cloning can be demonstrated after plasmid DNA isolation by control cleavage with the enzymes BgIII, BstEII and PvuII. The plasmid is called pTrc99AcsrA (figure 1).

### Example 2

### Preparation of L-threonine with the strain MG442/pTrc99AcsrA

The L-threonine-producing E. coli strain MG442 is described in the patent specification US-A-4,278,765 and deposited as CMIM B-1628 at the Russian National Collection for Industrial Microorganisms (VKPM, Moscow, Russia).

The strain MG442 is transformed with the expression plasmid pTrc99AcsrA described in example 1 and with the vector pTrc99A and plasmid-carrying cells are selected on LB agar with 50 µg/ml ampicillin. The strains MG442/pTrc99AcsrA and MG442/pTrc99A are formed in this manner. Selected individual colonies are then multiplied further on minimal medium with the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₉*7H₂O, 15 g/l CaCO₃, 20 g/l glucose, 50 mg/l ampicillin are inoculated and the batch is incubated for 16 hours at 37°C and 180 rpm on an ESR incubator from Kühner AG (Birsfelden, Switzerland).

250 µl portions of this preculture are transinoculated into 10 ml of production medium (25 g/l (NH₄)2SO₄, 2 g/l KH₂PO₄, 1 g/l MgSO₄*7H₂O 0.03 g/l FeSO₄*7H₂O, 0.018 g/l MnSO₄*1H₂O, 30 g/l CaCO₃, 20 g/l glucose, 50 mg/l ampicillin) and the batch is incubated for 48 hours at 37°C. The formation of L-threonine by the starting strain MG442 is investigated in the same manner, but no addition of ampicillin to the medium takes place. After the incubation the optical density (OD) of the culture suspension is determined with an LP2W photometer from Dr. Lange (Düsseldorf, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in table 1.

**Table 1**

| Strain | OD (660 nm) | L-Threonine g/l |
|---|---|---|
| MG442 | 5.6 | 1.4 |
| MG442/pTrc99A | 3.8 | 1.3 |
| MG442/pTrc99AcsrA | 4.2 | 1.8 |

### Brief Description of the Figure:

### Figure 1: Map of the plasmid pTrc99AcsrA containing the csrA gene.

The length data are to be understood as approx. data. The abbreviations and designations used have the following meaning:
- Amp: Ampicillin resistance gene
- lacI: Gene for the repressor protein of the trc promoter
- Ptrc: trc promoter region, IPTG-inducible
- csrA: Coding region of the csrA gene
- 5S: 5S rRNA region
- rrnBT: rRNA terminator region

The abbreviations for the restriction enzymes have the following meaning
- BglII: Restriction endonuclease from Bacillus globigii (ATCC 49760)
- BstEII: Restriction endonuclease from Bacillus stearothermophilus ATCC 12980
- HindIII: Restriction endonuclease from Haemophilus influenzae
- PvuII: Restriction endonuclease from Proteus vulgaris (ATCC 13315)
- XbaI: Restriction endonuclease from Xanthomonas campestris

## Claims

1. Process for the preparation of L-threonine, L-lysine, L-valine or L-homoserine, wherein microorganisms of the Enterobacteriaceae family which produce the desired L-amino acid and in which the csrA gene, which is the carbon storage regulator A, or nucleotide sequences coding for its gene product are over-expressed, are fermented.

2. Process according to claim 1, wherein, at the same time or optionally separately, the CsrA protein is over- expressed by elimination or reduced expression of the csrB gene.

3. Process according to claims 1 to 2, wherein the following further steps are carried out:
a) concentration of the desired L-amino acid in the medium or in the cells of the microorganisms, and
b) isolation of the desired L-amino acid, constituents of the fermentation broth and/or the biomass in its entirety or portions ( > 0 to100%) thereof optionally remaining in the product.

4. Process according to claim 1, wherein the expression of the polynucleotide which codes for the csrA gene is increased.

5. Process according to claim 1, wherein, for the preparation of L-threonine, microorganisms of the Enterobacteriaceae family are fermented in which additionally at the same time one or more of the genes chosen from the group consisting of:
5.1 the thrABC operon which codes for aspartate kinase, homoserine dehydrogenase, homoserine kinase and threonine synthase,
5.2 the pyc gene which codes for pyruvate carboxylase,
5.3 the pps gene which codes for phosphoenol pyruvate synthase,
5.4 the ppc genre which codes for phosphoenol pyruvate carboxylase,
5.5 the pntA and pntB genes which code for transhydrogenase,
5.6 the rhtB gene which imparts homoserine resistance,
5.7 the mqo gene which codes for malate:quinone oxidoreductase,
5.8 the rhtC gene which imparts threonine resistance,
5.9 the thrE gene which codes for the threonine export protein,
5.10 the gdhA gene which codes for glutamate dehydrogenase,
5.11 the hns gene which codes for the DNA-binding protein HLP-II,
5.12 the lrp gene which codes for the regulator of the leucine regulon,
5.13 the pgm gene which codes for phosphoglucomutase,
5.14 the fba gene which codes for fructose biphosphate aldolase,
5.15 the ptsH gene which codes for the phosphohistidine protein hexose phosphotransferase,
5.16 the ptsI gene which codes for enzyme I of the phosphotransferase system,
5.17 the crr gene which codes for the glucose-specific IIA component,
5.18 the ptsG gene which codes for the glucose-specific IIBC component,
5.19 the mopB gene which codes for 10 Kd chaperone,
5.20 the ahpC gene which codes for the small sub-unit of alkyl hydroperoxide reductase,
5.21 the ahpF gene which codes for the large sub-unit of alkyl hydroperoxide reductase,
5.22 the cysK gene which codes for cysteine synthase A,
5.23 the cysB gene which codes for the regulator of the cys regulon,
5.24 the cysJ gene which codes for the flavoprotein of NADPH sulfite reductase,
5.25 the cysI gene which codes for the haemoprotein of NADPH sulfite reductase,
5.26 the cysH gene which codes for adenylyl sulfate reductase,
5.27 the phoB gene which codes for the positive regulator PhoB of the pho regulon,
5.28 the phoR gene which codes for the sensor protein of the pho regulon,
5.29 the phoE gene which codes for protein E of outer cell membrane,
5.30 the pykF gene which codes for fructose-stimulated pyruvate kinase I,
5.31 the pfkB gene which codes for 6-phosphofructokinase II,
5.32 the malE gene which codes for the periplasmic binding protein of maltose transport,
5.33 the rseA gene which codes for a membrane protein with anti-sigmaE activity,
5.34 the rseC gene which codes for a global regulator of the sigmaE factor,
5.35 the sodA gene which codes for superoxide dismutase,
5.36 the sucA gene which codes for the decarboxylase sub-unit of 2-ketoglutarate dehydrogenase,
5.37 the sucB gene which codes for the dihydrolipoyltranssuccinase E2 sub-unit of 2-ketoglutarate dehydrogenase,
5.38 the sucC gene which codes for the β-sub-unit of succinyl-CoA synthetase,
5.39 the sucD gene which codes for the α-sub-unit of succinyl-CoA synthetase and
5.40 the iclR gene which codes for the regulator IclR of central intermediate metabolism is or are over-expressed.

6. Process according to claim 1, wherein, for the preparation of L-threonine, microorganisms of the Enterobacteriaceae family are fermented in which additionally at the same time one or more of the genes chosen from the group consisting of:
6.1 the tdh gene which codes for threonine dehydrogenase,
6.2 the mdh gene which codes for malate dehydrogenase,
6.3 the gene product of the open reading frame (orf) yjfA,
6.4 the gene product of the open reading frame (orf) ytfP,
6.5 the pckA gene which codes for phosphoenol pyruvate carboxykinase,
6.6 the poxB gene which codes for pyruvate oxidase,
6.7 the aceA gene which codes for isocitrate lyase,
6.8 the dgsA gene which codes for the DgsA regulator of the phosphotransferase system,
6.9 the fruR gene which codes for the fructose repressor,
6.10 the rpoS gene which codes for the sigma³⁸ factor,
6.11 the aspA gene which codes for aspartate ammonium lyase (aspartase) and
6.12 the aceB gene which codes for malate synthase A
is or are eliminated or reduced in expression.

7. A microorganism of the Enterobacteriaceae family producing L-threonine, L-lysine, L-valine or L-homoserine, in which the csrA gene or nucleotide sequences coding for its gene product are over-expressed compared to the starting microorganism.

## Patentansprüche

1. Verfahren zur Herstellung von L-Threonin, L-Lysin, L-Valin oder L-Homoserin, bei dem man Mikroorganismen der Enterobacteriaceae-Familie, die die gewünschte L-Aminosäure produzieren und bei denen das csrA-Gen, bei dem es sich um den Kohlenstoffspeicherregulator A (Carbon Storage Regulator A) handelt, oder Nukleotidsequenzen, die für dessen Genprodukt codieren, überexprimiert wird, fermentiert.

2. Verfahren nach Anspruch 1, wobei, gleichzeitig oder gegebenenfalls getrennt, das csrA-Protein durch Eliminieren oder eine verminderte Expression des csrB-Gens überexprimiert wird.

3. Verfahren nach Anspruch 1 oder 2, bei dem man die folgenden weiteren Schritte durchführt:
a) Konzentrieren der gewünschten L-Aminosäure im Medium oder in den Zellen des Mikroorganismus und
b) Isolieren der gewünschten L-Aminosäure, von Bestandteilen der Fermentationsbrühe und/oder der gesamten Biomasse oder Teilen (> 0 bis 100%) davon, die gegebenenfalls im Produkt verbleiben.

4. Verfahren nach Anspruch 1, bei dem man die Expression des Polynukleotids, das für das csrA-Gen codiert, erhöht.

5. Verfahren nach Anspruch 1, bei dem man, zur Herstellung von L-Threonin, Mikroorganismen der Enterobacteriaceae-Familie fermentiert, bei denen zusätzlich zur gleichen Zeit ein oder mehrere aus der aus:
5.1 dem thrABC-Operon, welches für Aspartatkinase, Homoserindehydrogenase, Homoserinkinase und Threoninsynthase codiert,
5.2 dem pyc-Gen, welches für Pyruvatcarboxylase codiert,
5.3 dem pps-Gen, welches für Phosphoenolpyruvatsynthase codiert,
5.4 dem ppc-Gen, welches für Phosphoenolpyruvatcarboxylase codiert,
5.5 den pntA- und pntB-Genen, die für Transhydrogenase codieren,
5.6 dem rhtB-Gen, welches Homoserinresistenz verleiht,
5.7 dem mqo-Gen, welches für Malat:Chinon-Oxidoreduktase codiert,
5.8 dem rhtC-Gen, welches Threoninresistenz verleiht,
5.9 dem thrE-Gen, welches für das Threoninexportprotein codiert,
5.10 dem gdhA-Gen, welches für Glutamatdehydrogenase codiert,
5.11 dem hns-Gen, welches für das DNA-bindende Protein HLP-II codiert,
5.12 dem lrp-Gen, welches für den Regulator des Leucinregulons codiert,
5.13 dem pgm-Gen, welches für Phosphoglucomutase codiert,
5.14 dem fba-Gen, welches für Fructosebiphosphataldolase codiert,
5.15 dem ptsH-Gen, welches für die Phosphohistidinproteinhexosephosphotransferas e codiert,
5.16 dem ptsI-Gen, welches für das Enzym I des Phosphotransferasesystems codiert,
5.17 dem crr-Gen, welches für die glucosespezifische IIA-Komponente codiert,
5.18 dem ptsG-Gen, welches für die glucosespezifische IIBC-Komponente codiert,
5.19 dem mopB-Gen, welches für das 10-kD-Chaperon codiert,
5.20 dem ahpC-Gen, welches für die kleine Untereinheit von Alkylhydroperoxidreduktase codiert,
5.21 dem ahpF-Gen, welches für die große Untereinheit von Alkylhydroperoxidreduktase codiert,
5.22 dem cysK-Gen, welches für Cysteinsynthase A codiert,
5.23 dem cysB-Gen, welches für den Regulator des cys-Regulons codiert,
5.24 dem cysJ-Gen, welches für das Flavoprotein von NADPH-Sulfitreduktase codiert,
5.25 dem cysI-Gen, welches für das Hämoprotein von NADPH-Sulfitreduktase codiert,
5.26 dem cysH-Gen, welches für Adenylylsulfatreduktase codiert,
5.27 dem phoB-Gen, welches für den positiven Regulator phoB des pho-Regulons codiert,
5.28 dem phoR-Gen, welches für das Sensorprotein des pho-Regulons codiert,
5.29 dem phoE-Gen, welches für das Protein E der äußeren Zellmembran codiert,
5.30 dem pykF-Gen, welches für fructosestimulierte Pyruvatkinase I codiert,
5.31 dem pfkB-Gen, welches für 6-Phosphofructokinase II codiert,
5.32 dem malE-Gen, welches für das periplasmatische Bindungsprotein des Maltosetransports codiert,
5.33 dem rseA-Gen, welches für ein Membranprotein mit anti-sigmaE-Aktivität codiert,
5.34 dem rseC-Gen, welches für einen globalen Regulator des sigmaE-Faktors codiert,
5.35 dem sodA-Gen, welches für Superoxiddismutase codiert,
5.36 dem sucA-Gen, welches für die Decarboxylase-Untereinheit von 2-Ketoglutaratdehydrogenase codiert,
5.37 dem sucB-Gen, welches für die Dihydrolipoyltranssuccinase-E2-Untereinheit von 2-Ketoglutaratdehydrogenase codiert,
5.38 dem sucC-Gen, welches für die β-Untereinheit von Succinyl-CoA-Synthetase codiert,
5.39 dem SucD-Gen, welches für die α-Untereinheit von Succinyl-CoA-Synthetase codiert und
5.40 dem iclR-Gen, welches für den Regulator IclR des zentralen Zwischenproduktmetabolismus codiert,
bestehenden Gruppe ausgewählte Gene überexprimiert werden.

6. Verfahren nach Anspruch 1, bei dem man, zur Herstellung von L-Threonin, Mikroorganismen der Enterobacteriaceae-Familie fermentiert, bei denen zusätzlich zur gleichen Zeit ein oder mehrere aus der aus:
6.1 dem tdh-Gen, welches für Threonindehydrogenase codiert,
6.2 dem mdh-Gen, welches für Malatdehydrogenase codiert,
6.3 dem Genprodukt des offenen Leserahmens (orf) yjfA,
6.4 dem Genprodukt des offenen Leserahmens (orf) ytfP,
6.5 dem pckA-Gen, welches für Phosphoenolpyruvatcarboxykinase codiert,
6.6 dem poxB-Gen, welches für Pyruvatoxidase codiert,
6.7 dem aceA-Gen, welches für Isocitratlyase codiert,
6.8 dem dgsA-Gen, welches für den DgsA-Regulator des Phosphotransferasesystems codiert,
6.9 dem fruR-Gen, welches für den Fructoserepressor codiert,
6.10 dem rpoS-Gen, welches für den sigma³⁸-Faktor codiert,
6.11 dem aspA-Gen, welches für Aspartatammoniumlyase (Aspartase) codiert und
6.12 dem aceB-Gen, welches für Malatsynthase A codiert,
bestehenden Gruppe ausgewählte Gene eliminiert sind oder eine verminderte Expression zeigen.

7. Mikroorganismus der Enterobacteriaceae-Familie, welches L-Threonin, L-Lysin, L-Valin oder L-Homoserin produziert, wobei, im Vergleich zum Ausgangsmikroorganismus, das csrA-Gen oder für dessen Genprodukt codierende Nukleotidsequenzen überexprimiert werden.

## Revendications

1. Procédé de préparation de L-thréonine, de L-lysine, de L-valine ou de L-homosérine, dans lequel on fermente des micro-organismes de la famille des Enterobacteriaceae qui produisent l'acide L-aminé souhaité, et dans lesquels le gène csrA, qui est le régulateur A du stockage du carbone, ou des séquences nucléotidiques codant pour son produit génique, sont surexprimés.

2. Procédé selon la revendication 1, dans lequel, en même temps ou en option séparément, la protéine CsrA est surexprimée par élimination ou expression réduite du gène csrB.

3. Procédé selon les revendications 1 à 2, dans lequel les étapes supplémentaires suivantes sont mises en oeuvre :
a) concentration de l'acide L-aminé souhaité dans le milieu ou dans les cellules des micro-organismes, et
b) isolement de l'acide L-aminé souhaité, de constituants du bouillon de fermentation et/ou de la biomasse dans leur totalité, ou dans des parties (> 0 à 100 %) de ces derniers, restant en option dans le produit.

4. Procédé selon la revendication 1, dans lequel l'expression du polynucléotide qui code pour le gène csrA est augmentée.

5. Procédé selon la revendication 1, dans lequel, pour la préparation de L-thréonine, des micro-organismes de la famille des Enterobacteriaceae sont fermentés, dans lesquels, en plus, et au même instant, un ou plusieurs des gènes choisis dans le groupe consistant en :
5.1 l'opéron thrABC, qui code pour l'aspartate kinase, l'homosérine déshydrogénase, l'homosérine kinase et la thréonine synthase,
5.2 le gène pyc, qui code pour la pyruvate carboxylase,
5.3 le gène pps, qui code pour la phosphoénol pyruvate synthase,
5.4 le gène ppc, qui code pour la phosphoénol pyruvate carboxylase,
5.5 les gènes pntA et pntB, qui codent pour la transhydrogénase,
5.6 le gène rhtB, qui confère une résistance à l'homosérine,
5.7 le gène mqo, qui code pour la malate:quinone oxydoréductase,
5.8 le gène rhtC, qui confère une résistance à la thréonine,
5.9 le gène thrE, qui code pour la protéine d'exportation de la thréonine,
5.10 le gène gdhA, qui code pour la glutamate déshydrogénase,
5.11 le gène hns, qui code pour la protéine HLP-II de liaison à l'ADN,
5.12 le gène lrp, qui code pour le régulateur du régulon de la leucine,
5.13 le gène pgm, qui code pour la phosphoglucomutase,
5.14 le gène fba, qui code pour la fructose biphosphate aldolase,
5.15 le gène ptsH, qui code pour la phosphohistidine protéine hexose phosphotransférase,
5.16 le gène ptsI, qui code pour l'enzyme I du système phosphotransférase,
5.17 le gène crr, qui code pour le composant IIA spécifique de glucose,
5.18 le gène ptsG, qui code pour le composant IIBC spécifique de glucose,
5.19 le gène mopB, qui code pour le chaperon 10 Kd,
5.20 le gène ahpC, qui code pour la petite sous-unité de l'alkyl hydroperoxyde réductase,
5.21 le gène ahpF, qui code pour la grande sous-unité de l'alkyl hydroperoxyde réductase,
5.22 le gène cysK, qui code pour la cystéine synthase A,
5.23 le gène cysB, qui code pour le régulateur du régulon cys,
5.24 le gène cysJ, qui code pour la flavoprotéine de la NADPH sulfite réductase,
5.25 le gène cysI, qui code pour l'hémoprotéine de la NADPH sulfite réductase,
5.26 le gène cysH, qui code pour l'adénylyl sulfate réductase,
5.27 le gène phoB, qui code pour le régulateur positif PhoB du régulon pho,
5.28 le gène phoR, qui code pour la protéine capteur du régulon pho,
5.29 le gène phoE, qui code pour la protéine E de la membrane cellulaire extérieure,
5.30 le gène pykF, qui code pour la pyruvate kinase I stimulée par le fructose,
5.31 le gène pfkB, qui code pour la 6-phosphofructokinase II,
5.32 le gène malE, qui code pour la protéine de liaison périplasmique du transport du maltose,
5.33 le gène rseA, qui code pour une protéine membranaire ayant une activité anti-sigmaE,
5.34 le gène rseC, qui code pour un régulateur global du facteur sigmaE,
5.35 le gène sodA, qui code pour la superoxyde dismutase,
5.36 le gène sucA, qui code pour la sous-unité décarboxylase de la 2-kétoglutaratedéshydrogénase,
5.37 le gène sucB, qui code pour la sous-unité dihydrolipoyltranssuccinase E2 de la 2-kétoglutaratedéshydrogénase,
5.38 le gène sucC, qui code pour la sous-unité β de la succinyl-CoA synthétase,
5.39 le gène sucD, qui code pour la sous-unité α de la succinyl-CoA synthétase, et
5.40 le gène iclR, qui code pour le régulateur IclR du métabolisme intermédiaire central,
sont surexprimés.

6. Procédé selon la revendication 1, dans lequel, pour la préparation de L-thréonine, des micro-organismes de la famille des Enterobacteriaceae sont fermentés, dans lesquels, en outre et au même instant, un ou plusieurs des gènes choisis dans le groupe consistant en :
6.1 le gène tdh, qui code pour la thréonine déshydrogénase,
6.2 le gène mdh, qui code pour la malate déshydrogénase,
6.3 le produit génique du cadre ouvert de lecture (orf) yjfA,
6.4 le produit génique du cadre ouvert de lecture (orf) ytfP,
6.5 le gène pckA, qui code pour la phosphoénol pyruvate carboxykinase,
6.6 le gène poxB, qui code pour la pyruvate oxydase,
6.7 le gène aceA, qui code pour l'isocitrate lyase,
6.8 le gène dgsA, qui code pour le régulateur DgsA du système phosphotransférase,
6.9 le gène fruR, qui code pour le répresseur du fructose,
6.10 le gène rpoS, qui code pour le facteur sigma³⁸,
6.11 le gène aspA, qui code pour l'aspartate ammonium lyase (aspartase), et
6.12 le gène aceB, qui code pour la malate synthase A, sont éliminés ou subissent une réduction de leur expression.

7. Micro-organisme de la famille des Enterobacteriaceae produisant la L-thréonine, la L-lysine, la L-valine ou la L-homosérine, dans lequel le gène csrA, ou des séquences nucléotidiques codant pour son produit génique, est surexprimé par rapport au micro-organisme de départ.


### SEQUENCE PROTOCOL

<110> Degussa AG
<120> Process for the preparation of non-aromatic L-amino acids using strains of the Enterobacteriaceae family
<130> 010457 BT
<160> 2
<170> PatentIn version 3.1
<210> 1
   <211> 20
   <212> DNA
   <213> artificial sequence
<220>
   <221> Primer
   <222> (1)..(20)
   <223> csrA1
<400> 1
   ctttcaagga gcaaagaatg 20
<210> 2
   <211> 21
   <212> DNA
   <213> artificial sequence
<220>
   <221> Primer
   <222> (1)..(21)
   <223> csrA2
<400> 2
   gatgagacgc ggaaagattag 21